# FASCICULE DE BREVET EUROPEEN

(11) **EP 0 858 440 B1**
(45) Date de publication et mention de la délivrance du brevet: **09.01.2002**
(21) Numéro de dépôt: 96934532.1
(22) Date de dépôt: 04.10.1996
(51) Int. Cl.: C07C 17/20, C07C 17/278, C07C 19/08, C07C 19/01

(54) **PROCEDE POUR LA PREPARATION DE 1,1,1,3,3-PENTAFLUOROPROPANE**
VERFAHREN ZUR HERSTELLUNG VON 1,1,1,3,3-PENTAFLUORPROPAN
METHOD FOR PREPARING 1,1,1,3,3-PENTAFLUOROPROPANE

(30) Priorité: 23.10.1995 FR 9512558
(43) Date de publication de la demande: 19.08.1998
(73) Titulaire: SOLVAY (Société Anonyme), 1050 Bruxelles (BE)
(72) Inventeur: WILMET, Vincent, B-1301 Wavre (BE); JANSSENS, Francine, B-1800 Vilvoorde (BE); SCHOEBRECHTS, Jean-Paul, B-5980 Grez-Doiceau (BE)
(74) Mandataire: Jacques, Philippe
(86) Numéro de dépôt international: EP9604315
(87) Numéro de publication internationale: WO9715540

(56) Documents cités:
- EP-A- 0 522 639
- EP-A- 0 703 205
- EP-A- 0 729 932
- WO-A-95/04021
- WO-A-96/01797

## Description

La présente invention se rapporte à un procédé pour la préparation de 1,1,1,3,3-pentafluoropropane (HFC-245fa). Elle concerne plus particulièrement un procédé de préparation du 1,1,1,3,3-pentafluoropropane au départ de 1,1,1,3,3-pentachloropropane.

Le 1,1,1,3,3-pentafluoropropane est un substitut possible des hydrocarbures chlorofluorés entièrement ou partiellement halogénés (CFCs et HCFCs), suspectés d'avoir un effet néfaste sur la couche d'ozone. Il s'avère notamment particulièrement intéressant comme agent gonflant pour la préparation de matériaux polymériques expansés.

Dans la demande WO 95/05353, on a proposé de préparer du 1,1,1,3,3-pentafluoropropane par réaction entre du 1,1-dichloro-2,2,2-trifluoroéthane (HCFC-123) et du dichlorodifluorométhane (CFC-12), suivi de l'hydrogénation du 1,1,1,3,3-pentafluoroprop-2-ène obtenu. Le rendement de la première étape de ce procédé connu (synthèse du 1,1,1,3,3-pentafluoroprop-2-ène intermédiaire) est toutefois très faible.

Dans la demande WO 95/04022, on a proposé de préparer du 1,1,1,3,3-pentafluoropropane par un procédé en trois étapes, consistant, dans une première étape, en la préparation de 1,1,1,3,3,3-hexachloropropane par réaction entre du tétrachlorométhane et du chlorure de vinylidène, dans une deuxième étape, en la conversion de l'hexachloropropane obtenu en du 1,1,1,3,3-pentafluoro-3-chloropropane par réaction avec du fluorure d'hydrogène et, dans une troisième étape, en la réduction du pentafluorochloropropane obtenu en 1,1,1,3,3-pentafluoropropane par réaction avec de l'hydrogène. Ce procédé présente le désavantage de générer des quantités importantes de 1,1,1,3,3,3-hexafluoropropane lors de la deuxième étape.

Dans la demande EP-A-611744, on a proposé de préparer du 1,1,1,3,3-pentafluoropropane par réaction entre du 1,1,1,3,3-pentafluoro-2,3-dichloropropane et de l'hydrogène. Le 1,1,1,3,3-pentafluoro-2,3-dichloropropane utilisé comme matière première dans ce procédé connu n'est toutefois pas un produit courant et il ne peut pas être aisément préparé.

La présente invention a pour but de fournir un procédé pour la préparation du 1,1,1,3,3-pentafluoropropane qui ne présente pas les inconvénients des procédés connus mentionnés ci-dessus, qui met en oeuvre des réactifs couramment ou aisément accessibles et qui présente un rendement élevé, répondant ainsi aux exigences économiques de l'industrie.

L'invention concerne dès lors un procédé pour la préparation de 1,1,1,3,3-pentafluoropropane, selon lequel on fait réagir du 1,1,1,3,3-pentachloropropane avec du fluorure d'hydrogène en présence d'un catalyseur d'hydrofluoration.

Les demandes de brevets WO 96/01797, EP 703 205 et EP 722 932 font partie de l'état de la technique en vertu de l'article 54(3) CBE.

Dans le procédé selon l'invention, le catalyseur d'hydrofluoration est avantageusement choisi parmi les dérivés des métaux des groupes 3, 4, 5, 13, 14 et 15 du tableau périodique des éléments (IUPAC 1988) et leurs mélanges (groupes du tableau périodique des éléments anciennement dénommés IIIa, IVa, IVb, Va, Vb et VIb). On entend par dérivés des métaux, les hydroxydes, les oxydes et les sels organiques et inorganiques de ces métaux ainsi que leurs mélanges. On retient particulièrement les dérivés du titane, du tantale, du molybdène, du bore, de l'étain et de l'antimoine. De préférence, le catalyseur est choisi parmi les dérivés des métaux des groupes 14 (IVa) et 15 (Va) du tableau périodique des éléments, et plus particulièrement parmi les dérivés de l'étain et de l'antimoine. Dans le procédé selon l'invention, les dérivés préférés des métaux sont les sels et ceux-ci sont de préférence choisis parmi les halogénures, et plus particulièrement parmi les chlorures, les fluorures et les chlorofluorures. Des catalyseurs d'hydrofluoration particulièrement préférés selon la présente invention sont les chlorures, les fluorures et les chlorofluorures d'étain et d'antimoine, notamment le tétrachlorure d'étain et le pentachlorure d'antimoine. Le pentachlorure d'antimoine est tout particulièrement recommandé.

Dans le cas où le catalyseur est sélectionné parmi les fluorures et les chlorofluorures métalliques, ceux-ci peuvent être obtenus au départ d'un chlorure que l'on soumet à une fluoration au moins partielle. Cette fluoration peut par exemple être réalisée au moyen de fluorure d'hydrogène, préalablement à la mise en contact du catalyseur avec le 1,1,1,3,3-pentachloropropane. En variante, elle peut être réalisée in situ, au cours de la réaction du 1,1,1,3,3-pentachloropropane avec le fluorure d'hydrogène.

La quantité de catalyseur mise en oeuvre peut varier dans de larges limites. Elle est généralement d'au moins 0,001 mole de catalyseur par mole de 1,1,1,3,3-pentachloropropane. De préférence, elle est d'au moins 0,01 mole de catalyseur par mole de 1,1,1,3,3-pentachloropropane. En principe, il n'y a pas de limite supérieure à la quantité de catalyseur mise en oeuvre. Par exemple, dans un procédé réalisé en continu en phase liquide, le rapport molaire entre le catalyseur et le 1,1,1,3,3-pentachloropropane peut atteindre 1000. En pratique toutefois, on utilise généralement au maximum environ 5 moles de catalyseur par mole de 1,1,1,3,3-pentachloropropane. De préférence, on ne dépasse pas environ 1 mole. De manière particulièrement préférée, on ne dépasse pas environ 0,5 mole de catalyseur par mole de 1,1,1,3,3-pentachloropropane.

Le rapport molaire entre le fluorure d'hydrogène et le 1,1,1,3,3-pentachloropropane mis en oeuvre est généralement d'au moins 5. De préférence, on travaille avec un rapport molaire d'au moins 8. Le rapport molaire entre le fluorure d'hydrogène et le 1,1,1,3,3-pentachloropropane mis en oeuvre ne dépasse en général pas 100. De préférence, il ne dépasse pas 50.

La température à laquelle s'effectue l'hydrofluoration est généralement d'au moins 50 °C. De préférence, elle est d'au moins 80 °C. La température ne dépasse en général pas 150 °C. De préférence, elle ne dépasse pas 130 °C. Avec du pentachlorure d'antimoine comme catalyseur, de bons résultats sont obtenus à une température de 100 à 120 °C.

Le procédé selon l'invention est de préférence réalisé en phase liquide. Dans ce cas, la pression est choisie de manière à maintenir le milieu réactionnel sous forme liquide. La pression mise en oeuvre varie en fonction de la température du milieu réactionnel. Elle est généralement de 2 bar à 40 bar. De manière préférée, on travaille à une température et à une pression auxquelles, en outre, le 1,1,1,3,3-pentafluoropropane produit est au moins partiellement sous forme gazeuse, ce qui permet de le séparer aisément du milieu réactionnel.

Le procédé selon l'invention peut être mis en oeuvre de manière continue ou discontinue. Il est entendu que la quantité de catalyseur mise en oeuvre est exprimée, dans un procédé discontinu, par rapport à la quantité initiale de 1,1,1,3,3-pentachloropropane mise en oeuvre et, dans un procédé continu, par rapport à la quantité stationnaire de 1,1,1,3,3-pentachloropropane présent dans la phase liquide.

Le temps de séjour des réactifs dans le réacteur doit être suffisant pour que la réaction du 1,1,1,3,3-pentachloropropane avec le fluorure d'hydrogène se réalise avec un rendement acceptable. Il peut être déterminé aisément en fonction des conditions opératoires retenues.

Le procédé selon l'invention peut être réalisé dans tout réacteur construit en un matériau résistant à la température, à la pression et aux réactifs utilisés, notamment au fluorure d'hydrogène. Il est avantageux de séparer du milieu réactionnel le 1,1,1,3,3-pentafluoropropane et le chlorure d'hydrogène au fur et à mesure qu'ils se forment et de maintenir ou de renvoyer dans le réacteur les réactifs non transformés, ainsi que les chlorofluoropropanes éventuellement formés par fluoration incomplète du 1,1,1,3,3-pentachloropropane. A cet effet, le procédé selon l'invention est avantageusement réalisé dans un réacteur équipé d'un dispositif de soutirage d'un courant gazeux, ce dispositif consistant par exemple en une colonne de distillation et un condenseur de reflux surmontant le réacteur. Ce dispositif permet, par un réglage adéquat, de soutirer en phase gazeuse le 1,1,1,3,3-pentafluoropropane et le chlorure d'hydrogène produits, tout en maintenant à l'état liquide dans le réacteur le 1,1,1,3,3-pentachloropropane et la majeure partie du fluorure d'hydrogène non transformés, ainsi que, le cas échéant, la majeure partie des produits de fluoration partielle du 1,1,1,3,3-pentachloropropane.

Le 1,1,1,3,3-pentachloropropane mis en oeuvre dans le procédé selon l'invention peut avantageusement être obtenu par réaction du chlorure de vinyle avec du tétrachlorométhane, comme décrit par exemple par Kotora M. et al, Journal of Molecular Catalysis, (1992), vol. 77, p. 51-60. Il est ainsi possible d'obtenir le 1,1,1,3,3-pentafluoropropane en deux étapes, au départ de produits aisément accessibles.

Dans une variante préférée, le procédé selon l'invention de préparation du 1,1,1,3,3-pentafluoropropane comprend une étape de télomérisation dans laquelle on fait réagir du chlorure de vinyle et du tétrachlorométhane en présence d'un catalyseur de télomérisation, de manière à obtenir du 1,1,1,3,3-pentachloropropane, et une étape ultérieure d'hydrofluoration dans laquelle on fait réagir le 1,1,1,3,3-pentachloropropane obtenu dans l'étape de télomérisation avec du fluorure d'hydrogène, en présence d'un catalyseur d'hydrofluoration.

Le catalyseur de télomérisation peut être choisi parmi les composés des métaux des groupes 8 à 11 du tableau périodique des éléments (IUPAC 1988) et leurs mélanges. Les composés des métaux des groupes 8 et 11 sont préférés. On retient particulièrement les composés du fer et du cuivre, ceux du cuivre étant tout particulièrement préférés. On entend par composés des métaux des groupes 8 à 11, les dérivés organiques et inorganiques de ces métaux ainsi que leurs mélanges. Les dérivés préférés sont les sels inorganiques, les chlorures étant particulièrement préférés. Des catalyseurs de télomérisation particulièrement préférés selon la présente invention sont le chlorure cuivreux, le chlorure cuivrique et leurs mélanges. De très bons résultats ont été obtenus avec le chlorure de cuivre (I) (chlorure cuivreux).

La quantité de catalyseur de télomérisation mise en oeuvre peut varier dans de larges limites. Elle est généralement d'au moins 0,001 mole de catalyseur par mole de chlorure de vinyle. De préférence, elle est d'au moins 0,005 mole de catalyseur par mole de chlorure de vinyle. Dans un procédé réalisé en continu en phase liquide, le rapport molaire entre le catalyseur et le chlorure de vinyle dans le milieu réactionnel peut atteindre 1000. Dans un procédé réalisé en discontinu, on utilise généralement au maximum environ 0,5 mole de catalyseur, de préférence pas plus de 0,2 mole de catalyseur et, de manière particulièrement préférée, 0,1 mole ou moins de catalyseur par mole de chlorure de vinyle mis en oeuvre.

Un co-catalyseur peut être mis en oeuvre dans l'étape de télomérisation. Des amines peuvent être utilisées comme co-catalyseur, de préférence à une concentration de 0,1 à 20 moles par mole de catalyseur de télomérisation. Comme amines utilisables comme co-catalyseur dans l'étape de télomérisation du procédé selon l'invention, on peut citer les alcanolamines, les alkylamines et les amines aromatiques, par exemple l'éthanolamine, la n-butylamine, la n-propylamine, l'isopropylamine, la benzylamine et la pyridine.

Le rapport molaire entre le tétrachlorométhane et le chlorure de vinyle mis en oeuvre dans l'étape de télomérisation est généralement d'au moins 1,5. De préférence, on travaille avec un rapport molaire d'au moins 2. En principe, il n'y a pas de limite supérieure au rapport molaire entre le tétrachlorométhane et le chlorure de vinyle. Par exemple, dans un procédé réalisé en continu en phase liquide, le rapport molaire entre les quantités stationnaires de tétrachlorométhane et de chlorure de vinyle dans le milieu réactionnel peut atteindre 1000. Dans un procédé réalisé en discontinu, on met généralement en oeuvre au maximum environ 50 moles, de préférence au maximum 20 moles et, de manière particulièrement préférée, au maximum 10 moles de tétrachlorométhane par mole de chlorure de vinyle.

La température à laquelle s'effectue la télomérisation du chlorure de vinyle par le tétrachlorométhane est généralement d'au moins 25 °C. De préférence, elle est d'au moins 70 °C. La température de télomérisation ne dépasse en général pas 200 °C. De préférence, elle ne dépasse pas 160 °C. Avec du chlorure cuivreux comme catalyseur, de bons résultats sont obtenus à une température de 100 à 140 °C, en particulier à une température de 110 à 130 °C.

La réaction de télomérisation est généralement réalisée en phase liquide, avantageusement en présence d'un solvant. Des solvants utilisables dans l'étape de télomérisation sont notamment des alcools, tels que le méthanol, l'éthanol, l'isopropanol et le tert-butanol et des nitriles, en particulier l'acétonitrile et le propionitrile. Les nitriles sont préférés. Le rapport molaire entre le solvant et le catalyseur de télomérisation ne dépasse en général pas 1000. De bons résultats ont été obtenus avec un rapport molaire entre le solvant et le catalyseur de télomérisation de 20 à 400.

Dans le procédé selon l'invention, la présence d'un nitrile est particulièrement avantageuse, spécialement lorsque le catalyseur de télomérisation est un chlorure, tout spécialement le chlorure cuivreux.

Les exemples ci-après illustrent l'invention de manière non limitative.

### Exemple 1 - Préparation du 1,1,1,3,3-pentachloropropane

Dans un autoclave de 1,5 l, revêtu d'une résine fluorocarbonée TEFLON®, équipé d'un agitateur mécanique et d'une sonde de température, on a introduit 4,43 moles d'acétonitrile, 6,57 moles de tétrachlorométhane, 0,11 mole de chlorure de cuivre (I) et 2,21 moles de chlorure de vinyle. L'autoclave a ensuite été plongé dans un bain thermostatisé, maintenu à une température de 120°C, pendant 66 h sous agitation continue. Après avoir atteint 8,5 bar, la pression autogène a diminué, atteignant 6 bar après 24 heures de réaction et 5,9 bar après 66 heures. L'autoclave a alors été refroidi, puis le milieu réactionnel a été distillé sous pression réduite. 380 g de 1,1,1,3,3-pentachloropropane ont été obtenus, ce qui représente un rendement de 80 % par rapport au chlorure de vinyle mis en oeuvre.

### Exemples 2-3 - Préparation du 1,1,1,3,3-pentachloropropane

Dans l'autoclave décrit à l'exemple 1, on a introduit de l'acétonitrile (AcN) du tétrachlorométhane, du chlorure de cuivre (I) et du chlorure de vinyle (VC) dans les proportions rapportées au tableau I. Les conditioins de réaction sous pression autogène et les résultats obtenus sont également présentés au tableau I.

**TABLE I**

| exemple | 2 | 3 |
|---|---|---|
| rapport molaire VC/CCl₄/AcN/CuCl | 1/6/2/0,07 | 1/3,1/2,2/0,03 |
| | | |
| Température de réaction | 120 °C | 115 °C |
| | | |
| Durée de réaction | 36 h | 96 h |
| | | |
| Conversion du VC (% de VC mis en oeuvre) | 83 % | 99 % |
| | | |
| Sélectivité en 1,1,1,3,3-pentachloropropane (% du VC converti transformé en 1,1,1,3,3-pentachloropropane) | 91 % | 85 % |

### Exemple 4 - hydrofluoration du 1,1,1,3,3-pentachloropropane

Dans un autoclave de 0,5 l en acier inoxydable HASTELLOY B2, équipé d'un agitateur mécanique à pales, d'une sonde de température et d'un tube plongeant permettant d'effectuer des prélèvements en phase liquide en cours d'essai, on a introduit 0,21 mole de 1,1,1,3,3-pentachloropropane, 0,076 mole de pentachlorure d'antimoine et 10 moles de fluorure d'hydrogène. L'autoclave a ensuite été plongé dans un bain thermostatisé, maintenu à une température de 120°C sous agitation continue pendant 21 heures. La pression a été régulée à 25 bar. Un prélèvement réalisé après 2 heures de réaction a montré que plus de 99 % molaire du 1,1,1,3,3-pentachloropropane mis en oeuvre était déjà transformé, dont 66 % en 1,1,1,3,3-pentafluoropropane. Après 21 heures de réaction, quasi tout le 1,1,1,3,3-pentachloropropane mis en oeuvre était transformé, dont 92 % molaire en 1,1,1,3,3-pentafluoropropane et environ 6 % en chlorofluoropropanes intermédiaires, formés par fluoration incomplète du 1,1,1,3,3-pentachloropropane.

## Revendications (Revendications pour l'(les) Etat(s) contractant(s) suivant(s): AT, CH, DK, GR, IE, LI, LU, MC, PT, SE)

1. Procédé pour la préparation de 1,1,1,3,3-pentafluoropropane selon lequel on fait réagir du 1,1,1,3,3-pentachloropropane avec du fluorure d'hydrogène en présence d'un catalyseur d'hydrofluoration et on sépare du milieu réactionnel le 1,1,1,3,3-pentafluoropropane et le chlorure d'hydrogène au fur et à mesure qu'ils se forment.

2. Procédé selon la revendication 1, dans lequel on effectue la réaction en continu dans une phase liquide où on maintient un rapport molaire entre le catalyseur et le 1,1,1,3,3-pentachloropropane de 0,001 à 1000.

3. Procédé selon la revendication 2, dans lequel on maintient un rapport molaire entre le catalyseur et le 1,1,1,3,3-pentachloropropane supérieur à 0,5.

4. Procédé selon une quelconque des revendications 1 à 3 dans lequel on effectue la réaction à une température et sous une pression auxquelles le 1,1,1,3,3-pentafluoropropane est gazeux et on soutire en phase gazeuse le 1,1,1,3,3-pentafluoropropane et le chlorure d'hydrogène.

5. Procédé selon une quelconque des revendications 1 à 4, dans lequel le catalyseur d'hydrofluoration est choisi parmi les dérivés du titane, du tantale, du molybdène, du bore, de l'étain et de l'antimoine.

6. Procédé selon une quelconque des revendications 1 à 5, dans lequel le catalyseur mis en oeuvre est le pentachlorure d'antimoine.

7. Procédé selon l'une quelconque des revendications 1 à 6, dans lequel on met en oeuvre de 5 à 100 moles de fluorure d'hydrogène par mole de 1,1,1,3,3-pentachloropropane.

8. Procédé selon l'une quelconque des revendications 1 à 7, dans lequel on réalise la réaction à une température de 50 à 150 °C et à une pression de 2 à 40 bar.

9. Procédé selon l'une quelconque des revendications 1 à 8, dans lequel le 1,1,1,3,3-pentachloropropane mis en oeuvre est préparé par réaction entre du chlorure de vinyle et du tétrachlorométhane.

10. Procédé pour la préparation de 1,1,1,3,3-pentafluoropropane selon lequel on fait réagir du 1,1,1,3,3-pentachloropropane avec du fluorure d'hydrogène en présence d'un catalyseur d'hydrofluoration choisi parmi les dérivés des métaux des groupes IIIa, IVa, IVb et VIb du tableau périodique des éléments et leurs mélanges.

11. Procédé selon la revendication 10 dans lequel le catalyseur d'hydrofluoration est choisi parmi les dérivés du titane, du molybdène, du bore et de l'étain

## Revendications (Revendications pour l'(les) Etat(s) contractant(s) suivant(s): FI)

1. Procédé pour la préparation de 1,1,1,3,3-pentafluoropropane selon lequel on fait réagir du 1,1,1,3,3-pentachloropropane avec du fluorure d'hydrogène en présence d'un catalyseur d'hydrofluoration.

2. Procédé selon la revendication 1, dans lequel on effectue la réaction en continu dans une phase liquide où on maintient un rapport molaire entre le catalyseur et le 1,1,1,3,3-pentachloropropane de 0,001 à 1000.

3. Procédé selon la revendication 2, dans lequel on maintient un rapport molaire entre le catalyseur et le 1,1,1,3,3-pentachloropropane supérieur à 0,5.

4. Procédé selon une quelconque des revendications 1 à 3 dans lequel on effectue la réaction à une température et sous une pression auxquelles le 1,1,1,3,3-pentafluoropropane est gazeux et on soutire en phase gazeuse le 1,1,1,3,3-pentafluoropropane et le chlorure d'hydrogène au fur et à mesure qu'ils se forment.

5. Procédé selon une quelconque des revendications 1 à 4, dans lequel le catalyseur d'hydrofluoration est choisi parmi les dérivés des métaux des groupes IIIa, IVa, IVb, Va, Vb et VIb du tableau périodique des éléments et leurs mélanges.

6. Procédé selon une quelconque des revendications 1 à 5, dans lequel le catalyseur d'hydrofluoration est choisi parmi les chlorures, les fluorures et les chlorofluorures d'étain et d'antimoine.

7. Procédé selon une quelconque des revendications 1 à 6, dans lequel le catalyseur mis en oeuvre est le pentachlorure d'antimoine.

8. Procédé selon l'une quelconque des revendications 1 à 7, dans lequel on met en oeuvre de 5 à 100 moles de fluorure d'hydrogène par mole de 1,1,1,3,3 -pentachloropropane.

9. Procédé selon l'une quelconque des revendications 1 à 8, dans lequel on réalise la réaction à une température de 50 à 150 °C et à une pression de 2 à 40 bar.

10. Procédé selon l'une quelconque des revendications 1 à 9, dans lequel le 1,1,1,3,3-pentachloropropane mis en oeuvre est préparé par réaction entre du chlorure de vinyle et du tétrachlorométhane.

## Revendications (Revendications pour l'(les) Etat(s) contractant(s) suivant(s): BE, DE, ES, FR, GB, IT, NL)

1. Procédé pour la préparation de 1,1,1,3,3-pentafluoropropane selon lequel on fait réagir du 1,1,1,3,3-pentachloropropane avec du fluorure d'hydrogène en présence d'un catalyseur d'hydrofluoration en continu dans une phase liquide où on maintient un rapport molaire entre le catalyseur et la quantité stationnaire de 1,1,1,3,3-pentachloropropane présent dans la phase liquide de 0,001 à 1000.

2. Procédé selon la revendication 1, dans lequel on maintient un rapport molaire entre le catalyseur et le 1,1,1,3,3-pentachloropropane supérieur à 0,5.

3. Procédé selon la revendication 1 ou 2 dans lequel on effectue la réaction à une température et sous une pression auxquelles le 1,1,1,3,3-pentafluoropropane est gazeux et on soutire en phase gazeuse le 1,1,1,3,3-pentafluoropropane et le chlorure d'hydrogène au fur et à mesure qu'ils se forment.

4. Procédé selon une quelconque des revendications 1 à 3, dans lequel le catalyseur d'hydrofluoration est choisi parmi les chlorures, les fluorures et les chlorofluorures d'étain et d'antimoine.

5. Procédé selon une quelconque des revendications 1 à 4, dans lequel le catalyseur mis en oeuvre est le pentachlorure d'antimoine.

6. Procédé selon l'une quelconque des revendications 1 à 5, dans lequel on met en oeuvre de 5 à 100 moles de fluorure d'hydrogène par mole de 1,1,1,3,3-pentachloropropane.

7. Procédé selon l'une quelconque des revendications 1 à 6, dans lequel on réalise la réaction à une température de 50 à 150 °C et à une pression de 2 à 40 bar.

8. Procédé selon l'une quelconque des revendications 1 à 7, dans lequel le 1,1,1,3,3-pentachloropropane mis en oeuvre est préparé par réaction entre du chlorure de vinyle et du tétrachlorométhane.

## Patentansprüche (Patentansprüche für folgende(n) Vertragsstaat(en): AT, CH, DK, GR, IE, LI, LU, MC, PT, SE)

1. Verfahren zur Herstellung von 1,1,1,3,3-Pentafluorpropan, wonach man 1,1,1,3,3-Pentachlorpropan in Anwesenheit eines Hydrofluorierungskatalysators mit Fluorwasserstoff umsetzt und aus dem Reaktionsmilieu das 1,1,1,3,3-Pentafluorpropan und den Chlorwasserstoff in dem Maße abtrennt, wie sie sich bilden.

2. Verfahren nach Anspruch 1, worin die Umsetzung kontinuierlich in einer flüssigen Phase vorgenommen wird, in der ein Molverhältnis zwischen dem Katalysator und dem 1,1,1,3,3-Pentachlorpropan von 0,001 bis 1.000 aufrechterhalten wird.

3. Verfahren nach Anspruch 2, worin ein Molverhältnis zwischen dem Katalysator und dem 1,1,1,3,3-Pentachlorpropan von über 0,5 aufrechterhalten wird.

4. Verfahren nach einem der Ansprüche 1 bis 3, worin die Umsetzung bei einer Temperatur und unter einem Druck ausgeführt wird, bei denen das 1,1,1,3,3-Pentafluorpropan gasförmig ist und das 1,1,1,3,3-Pentafluorpropan und der Chlorwasserstoff in gasförmiger Phase abgenommen werden.

5. Verfahren nach einem der Ansprüche 1 bis 4, worin der Hydroflurierungskatalysator unter den Derivaten von Titan, Tantal, Molybdän, Bor, Zinn und Antimon ausgewählt wird.

6. Verfahren nach einem der Ansprüche 1 bis 5, worin der eingesetzte Katalysator Antimonpentachlorid ist.

7. Verfahren nach einem der Ansprüche 1 bis 6, worin 5 bis 100 Mol Fluorwasserstoff pro Mol 1,1,1,3,3-Pentachlorpropan eingesetzt werden.

8. Verfahren nach einem der Ansprüche 1 bis 7, worin die Umsetzung bei einer Temperatur von etwa 50 bis 150°C und bei einem Druck von 2 bis 40 bar ausgeführt wird.

9. Verfahren nach einem der Ansprüche 1 bis 8, worin das eingesetzte 1,1,1,3,3-Pentachlorpropan durch Umsetzung von Vinylchlorid mit Tetrachlormethan hergestellt wird.

10. Verfahren zur Herstellung von 1,1,1,3,3-Pentafluorpropan, wonach man 1,1,1,3,3-Pentachlorpropan in Anwesenheit eines Hydrofluorierungskatalysators mit Fluorwasserstoff umsetzt, welcher Katalysator unter den derivaten von Metallen der Gruppen IIIa, IVa, IVb und VIb des Periodensystems der Elemente und unter deren Gemischen ausgewählt wird.

11. Verfahren nach Anspruch 10, worin der Hydrofluorierungskatalysator unter den Verbindungen von Titan, Molybdän, Bor und Zinn ausgewählt wird.

## Patentansprüche (Patentansprüche für folgende(n) Vertragsstaat(en): FI)

1. Verfahren zur Herstellung von 1,1,1,3,3-Pentafluorpropan, wonach man 1,1,1,3,3-Pentachlorpropan in Anwesenheit eines Hydrofluorierungskatalysators mit Fluorwasserstoff umsetzt.

2. erfahren nach Anspruch 1, worin die Umsetzung kontinuierlich in einer flüssigen Phase vorgenommen wird, in der man zwischen dem Katalysator und dem 1,1,1,3,3-Pentachlorpropan ein Molverhältnis von 0,001 bis 1000 aufrechterhält.

3. Verfahren nach Anspruch 2, worin man ein Molverhältnis zwischen dem Katalysator und dem 1,1,1,3,3-Pentachlorpropan von über 0,5 aufrechterhält.

4. Verfahren nach einem der Ansprüche 1 bis 3, worin die Umsetzung bei einer Temperatur und unter einem Druck ausgeführt wird, bei denen das 1,1,1,3,3-Pentafluorpropan gasförmig ist und das 1,1,1,3,3-Pentafluorpropan und der Chlorwasserstoff in der Gasphase in dem Maße abgenommen werden, wie sie sich bilden.

5. Verfahren nach einem der Ansprüche 1 bis 4, worin der Hydrofluorierungskatalysator unter den Derivaten von Metallen der Gruppen IIIa, IVa, IVb, Va, Vb und VIb des Periodensystems der Elemente und unter deren Gemischen ausgewählt wird.

6. Verfahren nach einem der Ansprüche 1 bis 5, worin der Hydrofluorierungskatalysator unter den Chloriden, Fluoriden und Chlorfluoriden von Zinn und Antimon ausgewählt wird.

7. Verfahren nach einem der Ansprüche 1 bis 6, worin der eingesetzte Katalysator das Antimonpentachlorid ist.

8. Verfahren nach einem der Ansprüche 1 bis 7, worin 5 bis 100 Mol Fluorwasserstoff pro Mol 1,1,1,3,3-Pentachlorpropan eingesetzt werden.

9. Verfahren nach einem der Ansprüche 1 bis 8, worin die Umsetzung bei einer Temperatur von ungefähr 50 bis 150°C und bei einem Druck von 2 bis 40 bar ausgeführt wird.

10. Verfahren nach einem der Ansprüche 1 bis 9, worin das eingesetzte 1,1,1,3,3-Pentachlorpropan durch Umsetzung von Vinylchlorid mit Tetrachlormethan hergestellt wird.

## Patentansprüche (Patentansprüche für folgende(n) Vertragsstaat(en): BE, DE, ES, FR, GB, IT, NL)

1. Verfahren zur Herstellung von 1,1,1,3,3-Pentafluorpropan, wonach man 1,1,1,3,3-Pentachlorpropan in Anwesenheit eines Hydrofluorierungskatalysators kontinuierlich in einer flüssigen Phase mit Fluorwasserstoff umsetzt, in welcher Phase ein Molverhältnis zwischen dem Katalysator und der in der flüssigen Phase vorliegenden stationären Menge an 1,1,1,3,3-Pentachlorpropan von 0,001 bis 1000 aufrechterhalten wird.

2. Verfahren nach Anspruch 1, worin ein Molverhältnis zwischen dem Katalysator und dem 1,1,1,3,3-Pentachlorpropan von über 0,5 aufrechterhalten wird.

3. Verfahren nach Anspruch 1 oder 2, worin die Umsetzung bei einer Temperatur und unter einem Druck ausgeführt wird, bei denen das 1,1,1,3,3-Pentafluorpropan gasförmig ist und das 1,1,1,3,3-Pentafluorpropan und der Chlorwasserstoff in der Gasphase in dem Maße abgezogen werden, wie sie sich bilden.

4. Verfahren nach einem der Ansprüche 1 bis 3, worin der Hydrofluorierungskatalysator unter den Chloriden, Fluoriden und den Chlorfluoriden von Zinn und Antimon ausgewählt wird.

5. Verfahren nach einem der Ansprüche 1 bis 4, worin der eingesetzte Katalysator das Antimonpentachlorid ist.

6. Verfahren nach einem der Ansprüche 1 bis 5, worin 5 bis 100 Mol Fluorwasserstoff pro Mol 1,1,1,3,3-Pentachlorpropan eingesetzt werden.

7. Verfahren nach einem der Ansprüche 1 bis 6, worin die Umsetzung bei einer Temperatur von etwa 50 bis 150°C und bei einem Druck von 2 bis 40 bar ausgeführt wird.

8. Verfahren nach einem der Ansprüche 1 bis 7, worin das eingesetzte 1,1,1,3,3-Pentachlorpropan durch Umsetzung von Vinylchlorid mit Tetrachlormethan hergestellt wird.

## Claims (Claims for the following Contracting State(s): AT, CH, DK, GR, IE, LI, LU, MC, PT, SE)

1. Process for the preparation of 1,1,1,3,3-pentafluoropropane, according to which 1,1,1,3,3-pentachloropropane is reacted with hydrogen fluoride in the presence of a hydrofluorination catalyst and the 1,1,1,3,3-pentafluoropropane and the hydrogen chloride are separated from the reaction mixture as they are being formed.

2. Process according to Claim 1, in which the reaction is carried out continuously in a liquid phase where a molar ratio of the catalyst to 1,1,1,3,3-pentachloropropane maintained from 0.001 to 1000.

3. Process according to claim 2, in which the molar ratio of the catalyst to 1,1,1,3,3-pentachloropropane is maintained superior to 0.5.

4. Process according to any one of Claims 1 at 3, in which the reaction is carried out at a temperature and under a pressure at which 1,1,1,3,3-pentafluoropropane is gaseous and in which 1,1,1,3,3-pentafluoropropane and hydrogen chloride are drawn off in a gaseous phase as they are being formed.

5. Process according to any one of Claims 1 to 4 in which the hydrofluorination catalyst is chosen from tin and antimony chlorides, fluorides and chlorofluorides.

6. Process according to any one of Claims 1 to 5, in which the catalyst used is antimony pentachloride.

7. Process according to any one of Claims 1 to 6, in which from 5 to 100 moles of hydrogen fluoride are used per mole of 1,1,1,3,3-pentachloropropane.

8. Process according to any one of Claims 1 to 7, in which the reaction is carried out at a temperature of approximately 50 to 150°C and at a pressure of 2 to 40 bar.

9. Process according to any one of Claims 1 to 8, in which the 1,1,1,3,3-pentachloropropane used is prepared by reaction between vinyl chloride and tetrachloromethane.

10. Process for the preparation of 1,1,1,3,3-pentafluoropropane according to which 1,1,1,3,3-pentachloropropane is reacted with hydrogen fluoride in the presence of a hydrofluorination catalyst selected from the derivatives of metals of group IIIa, IVa, IVb and VIb of the periodical table of elements and their mixtures.

11. Process according to claim 10, wherein the hydrofluorination catalyst is selected from the derivatives of titanium, molybdene, boron and tin.

## Claims (Claims for the following Contracting State(s): FI)

1. Process for the preparation of 1,1,1,3,3-pentafluoropropane, according to which 1,1,1,3,3-pentachloropropane is reacted with hydrogen fluoride in the presence of a hydrofluorination catalyst.

2. Process according to Claim 1, in which the reaction is carried out continuously in a liquid phase where a molar ratio of the catalyst to 1,1,1,3,3-pentachloropropane maintained from 0.001 to 1000.

3. Process according to claim 2, in which the molar ratio of the catalyst to 1,1,1,3,3-pentachloropropane is maintained superior to 0.5.

4. Process according to any one of Claims 1 at 2, in which the reaction is carried out at a temperature and under a pressure at which 1,1,1,3,3-pentafluoropropane is gaseous and in which 1,1,1,3,3-pentafluoropropane and hydrogen chloride are drawn off in a gaseous phase as they are being formed.

5. Process according to any one of Claims 1 to 4, wherein the hydrofluorination catalyst is selected from the derivatives of metals of groups IIIa, IVa, IVb, Va, Vb and VIb of the periodic table of the elements and their mixtures.

6. Process according to any one of Claims 1 to 5, in which the hydrofluorination catalyst is chosen from tin and antimony chlorides, fluorides and chlorofluorides.

7. Process according to any one of Claims 1 to 6, in which the catalyst used is antimony pentachloride.

8. Process according to any one of Claims 1 to 7, in which from 5 to 100 moles of hydrogen fluoride are used per mole of 1,1,1,3,3-pentachloropropane.

9. Process according to any one of Claims 1 to 8, in which the reaction is carried out at a temperature of approximately 50 to 150°C and at a pressure of 2 to 40 bar.

10. Process according to any one of Claims 1 to 9, in which the 1,1,1,3,3-pentachloropropane used is prepared by reaction between vinyl chloride and tetrachloromethane.

## Claims (Claims for the following Contracting State(s): BE, DE, ES, FR, GB, IT, NL)

1. Process for the preparation of 1,1,1,3,3-pentafluoropropane, according to which 1,1,1,3,3-pentachloropropane is reacted with hydrogen fluoride in the presence of a hydrofluorination catalyst, continuously in a liquid phase where a molar ratio of the catalyst to the stationary concentration of 1,1,1,3,3-pentachloropropane in the liquid phase from 0.001 to 1000 is maintained.

2. Process according to claim 1, in which the molar ratio of the catalyst to 1,1,1,3,3-pentachloropropane is maintained superior to 0.5.

3. Process according to Claim 1 or 2, in which the reaction is carried out at a temperature and under a pressure at which 1,1,1,3,3-pentafluoropropane is gaseous and in which 1,1,1,3,3-pentafluoropropane and hydrogen chloride are drawn off in a gaseous phase as they are being formed.

4. Process according to any one of Claims 1 to 3, in which the hydrofluorination catalyst is chosen from tin and antimony chlorides, fluorides and chlorofluorides.

5. Process according to any one of Claims 1 to 4, in which the catalyst used is antimony pentachloride.

6. Process according to any one of Claims 1 to 5, in which from 5 to 100 moles of hydrogen fluoride are used per mole of 1,1,1,3,3-pentachloropropane.

7. Process according to any one of Claims 1 to 6, in which the reaction is carried out at a temperature of approximately 50 to 150°C and at a pressure of 2 to 40 bar.

8. Process according to any one of Claims 1 to 7, in which the 1,1,1,3,3-pentachloropropane used is prepared by reaction between vinyl chloride and tetrachloromethane.
